# EUROPÄISCHE PATENTSCHRIFT

(11) **EP 1 403 242 B1**
(45) Veröffentlichungstag und Bekanntmachung des Hinweises auf die Patenterteilung: **15.11.2006**
(21) Anmeldenummer: 03020534.8
(22) Anmeldetag: 17.09.2003
(51) Int. Cl.: C07C 209/78, C07C 263/10, C08G 18/00

(54) **Verfahren zur Herstellung von Polyisocyanaten der Diphenylmethanreihe durch Phosgenierung von nicht-neutralisiertem Polyamin der Diphenylmethanreihe**
Process for the preparation of polyisocyanates of the diphenylmethane series by phosgenation of non-neutralised polyamines of the diphenylmethane series
Procédé pour la préparation de polyisocyanates de la série diphenylmethane par phosgenation de polyamines non-neutralisées de la serie diphenylmethane

(30) Priorität: 30.09.2002 DE 10245703
(43) Veröffentlichungstag der Anmeldung: 31.03.2004
(73) Patentinhaber: Bayer MaterialScience AG, 51368 Leverkusen (DE)
(72) Erfinder: Koch, Daniel, Dr., 47137 Duisburg (DE); Pirkl, Hans-Georg, Dr., 51377 Leverkusen (DE); Hagen, Torsten, Dr., 45133 Essen (DE); Wershofen, Stefan, Dr., 41065 Mönchengladbach (DE)

(56) Entgegenhaltungen:
- DE-A- 2 404 775
- GB-A- 1 203 546

## Beschreibung

Die Erfindung betrifft ein Verfahren zur Herstellung von Polyisocyanaten der Diphenylmethanreihe, welche durch Umsetzung der entsprechenden Polyamine der Diphenylmethanreihe mit Phosgen gewonnen werden.

Unter Polyisocyanaten der Diphenylmethanreihe werden Isocyanate und Isocyanatgemische folgenden Typs verstanden:

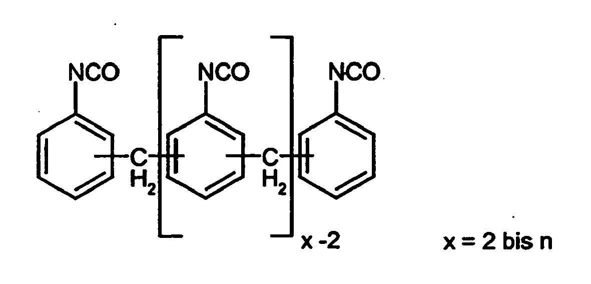

Analog werden unter Polyaminen der Diphenylmethanreihe Verbindungen und Verbindungsgemische folgenden Typs verstanden:

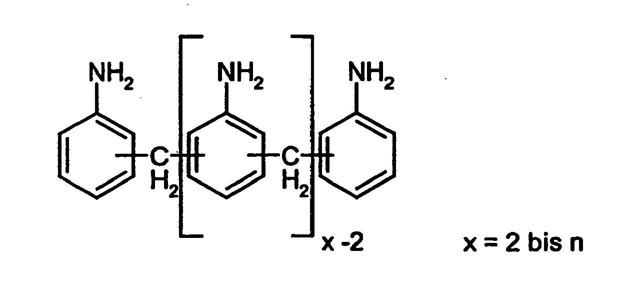

Die großtechnische Herstellung von Isocyanaten durch Umsetzung von Aminen mit Phosgen in Lösungsmitteln ist bekannt und in der Literatur ausführlich beschrieben (Ullmanns Enzyklopädie der technischen Chemie, 4. Auflage, Band 13, Seite 347-357, Verlag Chemie GmbH, Weinheim, 1977). Auf Basis dieses Verfahrens wird das Polyisocyanatgemisch hergestellt, das als Polyisocyanatkomponente bei der Herstellung von Polyurethanschäumen und anderen nach dem Polyadditionsverfahren hergestellten Polyurethankunststoffen dient.

Die kontinuierliche, diskontinuierliche oder halbkontinuierliche Herstellung von Polyaminen der Diphenylmethanreihe, nachfolgend auch als MDA bezeichnet, ist in zahlreichen Patenten und Publikationen beschrieben. Üblicherweise erfolgt die Herstellung durch Umsetzung von Anilin und Formaldehyd in Anwesenheit saurer Katalysatoren. Üblicherweise wird HCl als saurer Katalysator eingesetzt. Der saure Katalysator wird gemäß dem Stand der Technik zum Ende des Prozesses vor den abschließenden Aufarbeitungsschritten (wie beispielsweise der destillativen Entfernung von überschüssigem Anilin) durch Zusatz einer Base neutralisiert und somit verbraucht.

Hauptprodukte der säurekatalysierten Reaktion von Anilin und Formaldehyd sind das Diamin 4,4'-MDA, seine Stellungsisomere 2,4'-MDA und 2,2'-MDA sowie höhere homologe Polyamine der Diphenylmethanreihe. Die Polyisocyanate der Diphenylmethanreihe, nachfolgend als MDI bezeichnet, werden durch Phosgenierung der entsprechenden Polyamine hergestellt. Die so hergestellten Polyisocyanate der Diphenylmethanreihe enthalten dabei die verschiedenen Isocyanat-Isomeren und deren höhere Homologe in der gleichen Zusammensetzung wie die Polyamine, aus denen sie hergestellt wurden. Steuergröße zur Beeinflussung der Isomerenverteilung ist die bei der säurekatalysierten Reaktion von Anilin und Formaldehyd in den Prozess eingesetzte Menge an saurem Katalysator. Um MDI mit der gewünschten Isomerenverteilung herstellen zu können, müssen daher zum Teil erhebliche Mengen an saurem Katalysator und entsprechend erhebliche Mengen an Base für die Neutralisation des sauren Katalysators eingesetzt werden. Daraus ergeben sich weiterhin größere Mengen an salzhaltigen Abwasserströmen und entsprechend ein hoher Aufarbeitungs- und Entsorgungsaufwand.

Das Auffinden von Verfahren zur Umgehung oder Entschärfung dieser Problematik ist seit längerer Zeit das Ziel zahlreicher Versuche und Arbeiten, die in der Literatur beschrieben sind. So beschreibt beispielsweise WO-A1-0174755 die Herstellung von Polyaminen der Diphenylmethanreihe in Gegenwart von heterogenen Katalysatoren, welche die Funktion des sauren Katalysators übernehmen. Dieser Katalysatortyp kann im Gegensatz zu den üblicherweise eingesetzten Homogenkatalysatoren einfach vom Reaktionsgemisch abgetrennt werden und muss demnach vor der Aufarbeitung nicht neutralisiert werden. Nachteilig an diesem Verfahren ist aber, dass der saure Feststoff mit der Zeit desaktiviert, und dass das über diese Katalysatoren zugängliche Produktspektrum eingeschränkt ist. EP-A1-1167343 beschreibt die MDA-Herstellung gemäß dem Stand der Technik, erweitert um eine zusätzliche Abtrennung der 2,4'-MDA und 2,2'-MDA-Isomere, sowie deren Reaktion mit Formaldehyd und Rückführung dieses Gemisches an den Prozessanfang. Die rückgeführten Isomere werden somit bevorzugt zu höhermolekularen MDA-Bestandteilen umgesetzt. Hierdurch wird die enge Kopplung von Katalysatoreinsatz und Bildung von 2,4'- und 2,2'-MDA durch Einführung einer weiteren Steuergröße entschärft. Nachteilig an diesem Verfahren ist jedoch, dass die Umsetzung der rückgeführten Isomere zu höheren MDA-Homologen die Produkteigenschaften des durch anschließende Phosgenierung hergestellten MDI negativ beeinflussen kann, und dass für die Isomerentrennung zusätzlicher apparativer Aufwand für die Destillation erforderlich ist.

Aufgabe der vorliegenden Erfindung war es daher, ein technisches Verfahren zur Herstellung von Polyisocyanaten der Diphenylmethanreihe bereit zu stellen, mit dem der Verbrauch an saurem Katalysator und entsprechend von Base zur Neutralisation des sauren Katalysators auf der MDA-Stufe reduziert bzw. vermieden werden kann.

Die Aufgabe wird erfindungsgemäß gelöst durch ein Verfahren zur Herstellung von Polyisocyanaten der Diphenylmethanreihe, bei dem man
a) Anilin und Formaldehyd in Gegenwart von HCl zu einem Produktgemisch enthaltend Polyamine der Diphenylmethanreihe, HCl, Anilin und Wasser umsetzt, und danach
b) überschüssiges Anilin und Wasser durch Destillation in Gegenwast eines Schleppmittels entfernt, wobei man ein Produktgemisch enthaltend Polyamine der Diphenylmethanreihe, HCl sowie Anilin mit einem Gehalt von höchstens 10 Gew.-% bezogen auf die Polyamine und Wasser mit einem Gehalt von höchstens 5 Gew.-% bezogen auf die Polyamine erhält, und danach
c) das Produktgemisch enthaltend Polyamine der Diphenylmethanreihe, HCl sowie Anilin mit einem Gehalt von höchstens 10 Gew.-% bezogen auf die Polyamine und Wasser mit einem Gehalt von höchstens 5 Gew.-% bezogen auf die Polyamine phosgeniert.

Das Verfahren kann kontinuierlich, diskontinuierlich oder halbkontinuierlich durchgeführt werden. Mit dem Verfahren können Polyisocyanate der Diphenylmethanreihe ohne Neutralisation des sauren Katalysators HCl auf der Stufe der MDA-Herstellung hergestellt werden.

Das nach dem Verfahren in Schritt a) hergestellte Polyamin bzw. Polyamingemisch der Diphenylmethanreihe wird durch Kondensation von Anilin und Formaldehyd in Gegenwart des sauren Katalysators erhalten (H.J. Twitchett, Chem. Soc. Rev. 3(2), 209 (1974), M.V. Moore in: Kirk-Othmer Encycl. Chem. Technol., 3^{rd}. Ed., New York, 2, 338-348 (1978)). Für das erfindungsgemäße Verfahren ist es dabei unerheblich, ob Anilin und Formaldehyd zunächst in Abwesenheit von HCl vermischt werden und HCl anschließend zugesetzt wird, oder ob ein Gemisch aus Anilin und HCl mit Formaldehyd zu Reaktion gebracht wird.

Geeignete Polyamingemische der Diphenylmethanreihe werden üblicherweise erhalten durch Kondensation von Anilin und Formaldehyd im molaren Stoffinengenverhältnis 20:1 bis 1,6:1, bevorzugt 10:1 bis 1,8:1 sowie einem molaren Stoffmengenverhältnis von Anilin und HCl von 50:1 bis 1:1, bevorzugt 20:1 bis 2:1.

Formaldehyd wird technisch üblicherweise als wässrige Lösung eingesetzt. Es können jedoch auch andere methylengruppenliefernde Verbindungen wie z.B. Polyoxymethylenglykol, para-Formaldehyd oder Trioxan eingesetzt werden.

Als saure Katalysatoren zur MDA-Herstellung haben sich starke organische und vorzugsweise anorganische Säuren bewährt. Für das erfindungsgemäße Verfahren ist als saurer Katalysator ausschließlich HCl, bevorzugt in Form der wässrigen Lösung, geeignet.

In einer bevorzugten Ausführungsform des Verfahrens werden Anilin und HCl zunächst vereinigt. Dieses Gemisch wird, gegebenenfalls nach Abfuhr von Wärme, in einem weiteren Schritt mit Formaldehyd bei Temperaturen zwischen 20°C und 100°C, bevorzugt bei 30°C bis 70°C in geeigneter Weise vermischt und anschließend in einem geeigneten Verweilzeitapparat einer Vorreaktion unterzogen. Die Vorreaktion erfolgt bei Temperaturen zwischen 20°C bis 100°C, vorzugsweise im Temperaturbereich 30°C bis 80°C. Im Anschluss an Vermischung und Vorreaktion wird die Temperatur des Reaktionsgemisches in Stufen oder kontinuierlich und gegebenenfalls unter Überdruck auf 100°C bis 250°C, bevorzugt auf 100°C bis 180°C, besonders bevorzugt auf 100°C bis 160°C gebracht.

Es ist jedoch ebenfalls möglich, Anilin und Formaldehyd zunächst in Abwesenheit von HCl im Temperaturbereich von 5°C bis 130°C, bevorzugt von 40°C bis 110°C, besonders bevorzugt von 60°C bis 100°C, zu vermischen und so zur Reaktion zu bringen. Hierbei bilden sich Kondensationsprodukte aus Anilin und Formaldehyd (sog. Aminal). Im Anschluss an die Aminalbildung kann im Reaktionsgemisch vorhandenes Wasser durch Phasentrennung oder andere geeignete Verfahrensschritte, beispielsweise durch Destillation, entfernt werden. Das Kondensationsprodukt wird dann in einem weiteren Verfahrensschritt mit HCl in geeigneter Weise vermischt und in einem Verweilzeitapparat bei 20°C bis 100°C, bevorzugt 30°C bis 80°C einer Vorreaktion unterzogen. Anschließend wird die Temperatur des Reaktionsgemisches in Stufen oder kontinuierlich und ggf. unter Überdruck auf 100°C bis 250°C, bevorzugt auf 100°C bis 180°C, besonders bevorzugt auf 100°C bis 160°C gebracht.

Die Umsetzung von Anilin und Formaldehyd in Gegenwart von HCl zu Polyaminen der Diphenylmethanreihe kann in Anwesenheit weiterer Stoffe (z.B. Lösungsmittel, Salze, organische und anorganische Säuren) geschehen.

Das in Schritt a) erhaltene Produktgemisch enthält neben dem gewünschten MDA noch überschüssig eingesetztes Anilin, Wasser, HCl als Katalysator sowie gegebenenfalls weitere Stoffe, die dem Prozess zugesetzt wurden. Vor der Umsetzung dieser Mischung zum entsprechenden MDI mittels Phosgenierung muss dem Gemisch das überschüssige Anilin und das Wasser weitgehend entzogen werden. Dabei müssen Anilingehalte von höchstens 10 Gew.-%, bevorzugt von höchstens 2 Gew.-%, besonders bevorzugt von höchstens 0,2 Gew.-% bezogen auf die Polyamine und Wassergehalte von höchstens 5 Gew.-%, bevorzugt von höchstens 1 Gew.-%, besonders bevorzugt von höchstens 0,1 Gew.-% bezogen auf die Polyamine eingestellt werden.

Anilin muss dem in Schritt a) erhaltenen Produktgemisch vor der Phosgenierung weitgehend entzogen werden, da Anilin bei der Phosgenierung zu Phenylisocyanat umgesetzt würde. Phenylisocyanat ist aufgrund seiner kettenabbrechenden Monofunktionalität im MDI aber unerwünscht.

Die Entfernung von Wasser ist erforderlich, weil bei der Phosgenierung vorhandenes Wasser sowohl mit Phosgen als auch mit den Produkten und Zwischenprodukten der Phosgenierung reagieren würde, und somit eine verringerte. Ausbeute und unerwünschte Nebenprodukte im MDI resultieren würden.

Die Entfernung von Anilin in Schritt b) gelingt durch Destillation, obwohl das Anilin durch Reaktion mit HCl zumindest teilweise protoniert vorliegt. Vorzugsweise wird Wasser, welches ohnehin im Reaktionsgemisch vorhanden ist und ebenfalls entfernt werden muss, als Schleppmittel für die Entfernung des Anilins verwendet. Es ist aber auch möglich, andere organische oder anorganische Schleppmittel einzusetzen. Beispielsweise ist es möglich, als Schleppmittel das Lösungsmittel zu verwenden, welches auch in der Phosgenierung eingesetzt wird.

Die Abtrennung von Anilin und Wasser erfolgt vorteilhaft in der Weise, dass das saure Reaktionsgemisch der Anilin/Formaldehydkondensation einer Destillationskolonne zugeführt wird. Das Gemisch aus Anilin und Wasser und gegebenenfalls zusätzlichem Schleppmittel wird dabei als Leichtsiederfraktion am Kopf der Kolonne abgenommen. Das Anilin kann gegebenenfalls nach Aufarbeitung (beispielsweise durch Phasentrennung) in die MDA-Herstellung zurückgeführt werden. Das im Sumpf verbleibende Produktgemisch ist weitgehend anilin- und wasserfrei und enthält Anilin mit Gehalten von höchstens 10,0 Gew.-% bezogen auf die enthaltenen Polyamine und Wasser mit Gehalten von höchstens 5,0 Gew.-% bezogen auf die enthaltenen Polyamine. Um die gewünschten niedrigen Anilingehalte einzustellen, ist eine zusätzliche Zufuhr von Wasser und/oder anderem Schleppmittel erforderlich. Dies kann z.B. dadurch geschehen, dass dem in die Destillationsstufe einlaufenden Material eine entsprechende Wassermenge und/oder Lösungsmittelmenge zugeführt wird. Es ist aber auch möglich, die erforderliche Wasser- bzw. Lösungsmittelmenge dampfförmig in die Destillationsstufe einzubringen und damit gleichzeitig die für die Destillation nötige Energie einzutragen.

Es ist ebenfalls möglich, die Abtrennung von Anilin und Wasser mehrstufig, vorzugsweise zweistufig durchzuführen. Dabei kann zunächst die Abtrennung von Anilin durch Destillation mit Wasser als Schleppmittel erfolgen und dann in einer zweiten Destillationsstufe die Wasserabtrennung unter Einsatz eines anderen Schleppmittels durchgeführt werden.

Dabei müssen Anilingehalte von höchstens 10 Gew.-%, bevorzugt von höchstens 2 Gew.-%, besonders bevorzugt von höchstens 0,2 Gew.-% bezogen auf die Polyamine und Wassergehalte von höchstens 5 Gew.-%, bevorzugt von höchstens 1 Gew.-%, besonders bevorzugt von höchstens 0,1 Gew.-% bezogen auf die Polyamine eingestellt werden.

Das so erhaltene Produktgemisch enthält Polyamine der Diphenylmethanreihe und HCl als Hauptkomponenten sowie ggf. Restgehalte an Anilin und Wasser, wobei die Polyamine und ggf. Anilin teilweise in protonierter Form vorliegen. Dennoch kann dieses Produktgemisch anschließend mit Phosgen in einem inerten organischen Lösungsmittel zu den entsprechenden Isocyanaten umgesetzt werden. Das Molverhältnis von Polyamin zu Phosgen wird zweckmäßigerweise so bemessen, dass pro Mol Aminfunktion im Polyamin 1 bis 10 Mol, vorzugsweise 1,2 bis 6 Mol Phosgen eingesetzt werden. Als inerte Lösungsmittel für den Phosgenierschritt haben sich chlorierte, aromatische Kohlenwasserstoffe wie z.B. Monochlorbenzol, Dichlorbenzole, Trichlorbenzole, die entsprechenden (ggf. chlorierten) Toluole und Xylole sowie Chlorethylbenzol bewährt. Insbesondere finden Monochlorbenzol, Dichlorbenzol oder Mischungen dieser Chlorbenzole als inerte organische Lösungsmittel Anwendung. Die Menge an Lösungsmittel wird vorzugsweise so bemessen, dass die Reaktionsmischung einen MDI-Gehalt von 2 bis 50 Gew.-%, vorzugsweise zwischen 5 und 30 Gew.-%, bezogen auf das Gesamtgewicht der Reaktionsmischung, aufweist. Nach Beendigung der Umsetzung von Amin und Phosgen werden aus der Reaktionsmischung überschüssiges Phosgen, inerte organische Lösungsmittel und HCl abgetrennt. Dabei setzt sich das abgetrennte HCl aus dem bei der Phosgenierung von MDA mit Phosgen gebildeten HCl und dem als Katalysator für die Umsetzung von Anilin mit Formaldehyd zu MDA eingesetzten HCl zusammen. Als Produkt wird MDI erhalten, welches weiteren Aufarbeitungsschritten unterzogen werden kann.

Das erfindungsgemäße Verfahren hat den Vorteil, dass es auf den Einsatz einer Base wie NaOH zur Neutralisation des eingesetzten HCl verzichten kann. Hierdurch werden salzhaltige Abwasserströme und der damit verbundene Aufarbeitungs- und Entsorgungsaufwand vermieden. Das bei der Kondensation von Anilin und Formaldehyd als Katalysator eingesetzte HCl wird bei der Phosgenierung als Wertstoff zurückgewonnen und kann nach entsprechender Aufarbeitung in den MDA-Prozess zurückgeführt werden. Weiterhin kann auf einen Neutralisations- und Wäscheschritt im MDA-Prozess verzichtet werden.

### Beispiel

Zu 513 g Anilin wurden bei 80°C innerhalb von 20 min zugleich 884 g Anilin und 486 g einer 32%igen wässrigen Formaldehydlösung getropft. Nach der Zugabe wurde noch 10 min gerührt und anschließend eine Phasentrennung bei 70-80°C vorgenommen. Von der organischen Phase wurde eine Menge von 356 g auf 35°C temperiert und anschließend bei dieser Temperatur innerhalb von 30 min mit der restlichen organischen Phase und 427 g einer 32%igen wässrigen Salzsäure versetzt. Nach beendeter Zugabe und einer 30 minütigen Nachrührzeit bei dieser Temperatur wurde während 10 min auf 60°C aufgeheizt und 30 min bei dieser Temperatur gehalten. Anschließend wurde innerhalb von 30 min auf Rückflusstemperatur aufgeheizt und 10 h unter Rückfluss gerührt.

1177 g des hergestellten sauren Kondensationsgemisches wurden in eine diskontinuierliche Destillationsapparatur überführt und mittels Einblasen von Wasserdampf in den Sumpf auf einen Anilingehalt im Sumpf von unter 0,1 Gew.-% bezogen auf Polyamin gebracht. Das so erhaltene, weitgehend anilinfreie, aber noch wasser- und HCl-haltige Sumpfgemisch wurde in einer zweiten Destillationsapparatur so lange kontinuierlich mit Chlorbenzol versetzt und unter Sieden gehalten, bis der Wassergehalt im Sumpf unter 0.1 Gew.-% bezogen auf Polyamin abfiel. Das am Kopf kondensierende Gemisch aus Chlorbenzol und Wasser kann durch Phasentrennung getrennt werden, um das Chlorbenzol in die Destillation zurückzuführen.

Das nunmehr als Suspension in Chlorbenzol vorliegende, weitgehend anilin- und wasserfreie saure Umlagerungsgemisch der Anilin/Formaldehydkondensation wurde aus der Destillationsapparatur entnommen. Anschließend wurde Chlorbenzol zugegeben und ein Gehalt an Polyamin von 16 Gew.-% bezogen auf die Suspension eingestellt.

Von dieser Suspension wurden 300 g auf 55°C erwärmt und rasch und unter intensivem Rühren zu einer auf 0°C temperierten Lösung von 105 g Phosgen in 310 ml Chlorbenzol gegeben. Das resultierende Reaktionsgemisch wurde unter Durchleiten von Phosgen innerhalb von 45 min auf 100°C und anschließend während 10 min auf Rückflusstemperatur aufgeheizt. Nach weiteren 10 min bei dieser Temperatur wurde das Chlorbenzol unter vermindertem Druck bis zu einer Sumpftemperatur von 100°C abdestilliert. Das klare Roh-Isocyanat wurde anschließend in einer Destillationsapparatur bei einem Druck von 4-6 mbar durch ein auf 260°C erwärmtes Heizbad bis zum beginnenden Produktübergang erhitzt und daraufhin innerhalb von 5 min auf Raumtemperatur abgekühlt.

Das erhaltende MDI weist einen NCO-Gehalt von 32,5 Gew.-% bezogen auf MDI auf.

## Patentansprüche

1. Verfahren zur Herstellung von Polyisocyanaten der Diphenylmethanreihe, bei dem man
a) Anilin und Formaldehyd in Gegenwart von HCl zu einem Produktgemisch enthaltend Polyamine der Diphenylmethanreihe, HCl, Anilin und Wasser umsetzt, und danach
b) überschüssiges Anilin und Wasser durch Destillation in Gegenwast eines Schleppmittels entfernt, wobei man ein Produktgemisch enthaltend Polyamine der Diphenylmethanreihe, HCl sowie Anilin mit einem Gehalt von höchstens 10 Gew.-% bezogen auf die Polyamine und Wasser mit einem Gehalt von höchstens 5 Gew.-% bezogen auf die Polyamine erhält, und danach
c) das Produktgemisch enthaltend Polyamine der Diphenylmethanreihe, HCl sowie Anilin mit einem Gehalt von höchstens 10 Gew.-% bezogen auf die Polyamine und Wasser mit einem Gehalt von höchstens 5 Gew.-% bezogen auf die Polyamine phosgeniert.

2. Verfahren nach Anspruch 1, bei dem man die Destillation zweistufig durchführt, wobei man in einer ersten Destillationsstufe Anilin in Gegenwart von Wasser als Schleppmittel entfernt und in einer zweiten Destillationsstufe Wasser entfernt.

3. Verfahren nach einem der Ansprüche 1 bis 2, bei dem man in Schritt b) ein Produktgemisch enthaltend Polyamine der Diphenylmethanreihe, HCl sowie Anilin mit Gehalten von höchstens 2 Gew.-% bezogen auf die Polyamine und Wasser mit einem Gehalt von höchstens 1 Gew.-% bezogen auf die Polyamine erhält und danach in Schritt c) phosgeniert.

4. Verfahren nach einem der Ansprüche 1 bis 3, bei dem man in Schritt b) ein Produktgemisch enthaltend Polyamine der Diphenylmethanreihe, HCl sowie Anilin mit Gehalten von höchstens 0,2 Gew.-% bezogen auf die Polyamine und Wasser mit einem Gehalt von höchstens 0,1 Gew.-% bezogen auf die Polyamine erhält und danach in Schritt c) phosgeniert.

## Claims

1. Process for the production of polyisocyanates of the diphenylmethane series, in which
a) aniline and formaldehyde are reacted in the presence of HCl to give a product mixture containing polyamines of the diphenylmethane series, HCl, aniline and water, and then
b) excess aniline and water are removed by distillation in the presence of an entrainer, a product mixture containing polyamines of the diphenylmethane series, HCl and aniline with a content of no more than 10 wt.%, based on the polyamines, and water with a content of no more than 5 wt.%, based on the polyamines, being obtained, and then
c) the product mixture containing polyamines of the diphenylmethane series, HCl and aniline with a content of no more than 10 wt.%, based on the polyamines, and water with a content of no more than 5 wt.%, based on the polyamines, is phosgenated.

2. Process according to claim 1, wherein the distillation is performed in two steps, aniline being removed in the presence of water as entrainer in a first distillation step and water being removed in a second distillation step.

3. Process according to one of claims 1 to 2, wherein, in step b), a product mixture containing polyamines of the diphenylmethane series, HCl and aniline with contents of no more than 2 wt.%, based on the polyamines, and water with a content of no more than 1 wt.%, based on the polyamines, is obtained and then phosgenated in step c).

4. Process according to one of claims 1 to 3, wherein, in step b), a product mixture containing polyamines of the diphenylmethane series, HCl and aniline with contents of no more than 0.2 wt.%, based on the polyamines, and water with a content of no more than 0.1 wt.%, based on the polyamines, is obtained and then phosgenated in step c).

## Revendications

1. Procédé de fabrication de polyisocyanates de la série diphénylméthane, pour lequel
a) on met en réaction l'aniline et le formaldéhyde en présence d'HCl pour obtenir un mélange de produits contenant des polyamines de la série diphénylméthane, HCl, l'aniline et de l'eau, et ensuite
b) on élimine l'aniline et l'eau excédentaire par distillation en présence d'un agent d'entraînement, de façon à obtenir un mélange de produits contenant des polyamines de la série diphénylméthane, HCl ainsi que de l'aniline avec une teneur de maximum 10 % en poids rapportée aux polyamines et de l'eau avec une teneur de maximum 5 % en poids rapportée aux polyamines, et ensuite
c) on met en réaction par phosgénation le mélange de produits contenant des polyamines de la série diphénylméthane, HCl ainsi que de l'aniline avec une teneur de maximum 10 % en poids rapportée aux polyamines et de l'eau avec une teneur de maximum 5 % en poids rapportée aux polyamines.

2. Procédé suivant la revendication 1, dans lequel on effectue la distillation en deux étapes, l'aniline étant éliminée en présence d'eau comme agent d'entraînement dans une première étape et l'eau étant éliminée dans une deuxième étape de distillation.

3. Procédé suivant l'une des revendications 1 à 2, dans lequel on obtient à l'étape b) un mélange de produits contenant des polyamines de la série diphénylméthane, HCl ainsi que de l'aniline avec des teneurs de maximum 2 % en poids rapportée aux polyamines et de l'eau avec une teneur de maximum 1 % en poids rapportée aux polyamines et on le met ensuite en réaction par phosgénation à l'étape c).

4. Procédé suivant l'une des revendications 1 à 3, dans lequel on obtient à l'étape b) un mélange de produits contenant des polyamines de la série diphénylméthane, HCl ainsi que de l'aniline avec des teneurs de maximum 0,2 % en poids rapportée aux polyamines et de l'eau avec une teneur de maximum 0,1 % en poids rapportée aux polyamines et on le met ensuite en réaction par phosgénation à l'étape c).
